(19) Europäisches Patentamt
European Patent Office
Office européen des brevets

(11) **EP 4 410 829 A1**

(12) **EUROPEAN PATENT APPLICATION**
published in accordance with Art. 153(4) EPC

(43) Date of publication:
**07.08.2024 Bulletin 2024/32**

(21) Application number: **22873045.3**

(22) Date of filing: **27.09.2022**

(51) International Patent Classification (IPC):
*C07K 16/00* (2006.01)          *C12N 15/09* (2006.01)
*C12N 15/10* (2006.01)          *C12P 21/00* (2006.01)
*C12P 21/08* (2006.01)

(52) Cooperative Patent Classification (CPC):
**C07K 16/00; C12N 9/00; C12N 15/09; C12N 15/10; C12P 21/00**

(86) International application number:
**PCT/JP2022/035925**

(87) International publication number:
**WO 2023/048294 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.09.2021 PCT/JP2021/035224**

(71) Applicants:
• **Revolka Ltd.**
**Tokyo, 113-0033 (JP)**
• **Tohoku University**
**Sendai-shi, Miyagi 980-8577 (JP)**

(72) Inventors:
• **NAKAZAWA, Hikaru**
**Sendai-shi, Miyagi 980-8577 (JP)**
• **ITO, Tomoyuki**
**Sendai-shi, Miyagi 980-8577 (JP)**

• **KURIHARA, Daichi**
**Sendai-shi, Miyagi 980-8577 (JP)**
• **KAWADA, Sakiya**
**Sendai-shi, Miyagi 980-8577 (JP)**
• **UMETSU, Mitsuo**
**Sendai-shi, Miyagi 980-8577 (JP)**
• **KATAOKA, Shiro**
**Tokyo 103-0012 (JP)**
• **YAMAZAKI, Ryo**
**Tokyo 103-0012 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **MACHINE LEARNING-BASED PROTEIN DESIGN METHOD**

(57)     The present invention relates to a method of producing a protein for which two or more characteristics are optimized simultaneously. More specifically, the present invention relates to a method of producing a protein for which two or more characteristics are optimized, the method comprising: 1) providing a library comprising mutants from random mutation of a target protein; 2) determining respective characteristic values that indicate the two or more characteristics of some of the mutants in the library, and scoring the two or more characteristic values as one value per mutant by normalizing and integrating the characteristic values; 3) conducting machine learning by using the score values and ranking the library; and 4) selecting a protein for which two or more characteristics are optimized, based on the ranking results, wherein the two or more characteristic values are numerical values based on different measurement data related to respective different characteristics.

EP 4 410 829 A1

**Description**

Technical Field

Related Application

The present application claims priority to International Application No. PCT/JP2021/035224 (filed on September 27, 2021), the contents of which are incorporated herein by reference.

Field of the Invention

The present invention relates to a machine learning-based protein production method. More specifically, the present invention relates to a method of producing a protein for which two or more characteristics based on different measurement data are optimized.

Background Art

There has been a great need to modify functional proteins such as antibodies and enzymes to improve the functions thereof. Recently, studies have been conducted to more efficiently modify the functions of proteins by using machine learning. In these studies, a mutant library is made in a certain scale, and amino acid sequences and functions of mutants are experimentally evaluated to obtain data linked therewith, and then the data is used as training data to construct a machine learning model for predicting functions from sequences. Accordingly, the constructed machine learning model is used to predict mutants which are expected to improve the functions.

Regarding datasets for machine learning, two datasets: a direct linked dataset and an indirect linked dataset of amino acid sequences with values of functions and physical properties, are applied. The direct linked dataset is a set of data of which values of the functions and physical properties of each mutant are measured and linked to a corresponding sequence of the mutant (Non Patent Literature 1 etc.). On the other hand, for the indirect linked dataset, values of the functions and physical properties are not directly measured, and the read counts for amino acid sequences by deep sequence analysis or the like are used as alternatives to the values of the functions and physical properties to create a dataset (Non Patent Literatures 2 and 3).

The direct linking between amino acid sequences and values of the functions and physical properties has a possibility to be a high-quality dataset for machine learning; however, generating a large-sized dataset is difficult, only tens to hundreds of datasets can be generated, and sequences for exploring are also limited. On the other hand, although the quality of the indirect linked dataset is lower than that of the direct linked dataset, larger-sized data of amino acid sequences that can be obtained by deep sequence analysis can be used. Therefore, when the positions and the number of mutant residues and expressed amino acids are limited, the direct linked dataset is applied, and for discovery for antibody lead molecules by a molecule presentation method, the indirect linked dataset is often applied.

One of the interesting points of machine learning is that a solution can be derived in complex phenomena where the simultaneous solution across multiple conditions is required (multi-task machine learning). Prediction of amino acid sequences that optimize a plurality of functions and physical properties of proteins in a simultaneous process needs to link the amino acid sequences with respective values of the functions and physical properties. However, the values of the various functions and physical properties are difficult to express from the deep sequence analysis indirectly, and the direct linked dataset is therefore desirably used in which a measurement method is suitable to obtain respective values of the functions and physical properties. Nevertheless, a plurality of different functions and physical properties based on different measurement data are often contradictory to each other (e.g., antigen-binding functions versus expression levels and heat resistance of an antibody), and there are often cases where a plurality of functions and physical properties do not have common factors or useful characteristics within a target spatial sequence that can be handled by the direct linked dataset. For that, an approach has been proposed in which machine learning is performed on each single value of a function and physical property by direct linking, and then the obtained results are combined to improve a plurality of functions and physical properties (Patent Literatures 1 and 2).

The present inventors have confirmed that the following characteristics based on one piece of measurement data of fluorescence spectrum: fluorescence intensity and yellow fluorescence ratio, can be simultaneously optimized (Non Patent Literature 1 cited above). However, as mentioned above, a plurality of functions and physical properties based on different measurement data are often contradictory to each other, and hence, no approach has yet been reported in which a plurality of characteristics based on different measurement data can be optimized in a simultaneous process.

Citation List

Patent Literature

Patent Literature 1: WO2005/012877
Patent Literature 2: WO2005/013090

Non Patent Literature

Non Patent Literature 1: Saito et al., "Machine-Learning-Guided Mutagenesis for Directed Evolution of Fluorescent Proteins" ACS Synth Biol. 2018;7(9):2014-2022
Non Patent Literature 2: Liu et al., "Antibody complementarity determining region design using high-capacity machine learning" Bioinformatics, 2020;36 (7) :2126-2133
Non Patent Literature 3: Saka et al., "Antibody design using LSTM based deep generative model from phage display library for affinity maturation" Scientific Reports, 2021;11(1):5852

Summary of Invention

Technical Problem

An object of the present invention is to provide a protein design (production) method in which two or more characteristics based on different measurement data related to respective different characteristics are optimized in a simultaneous process.

Solution to Problem

The present inventors have evaluated two or more characteristics on some mutants in a mutant library prepared by random mutation, scored the two or more characteristics as one value per mutant, and performed machine learning by using the score as training data, and thereby succeeded in optimizing two or more characteristics in a simultaneous process to predict amino acid sequences.

That is, the present invention relates to the following 1) to 16).

[1] A method of producing a protein for which two or more characteristics are optimized, comprising:

1) providing a library comprising mutants from random mutation of a target protein;
2) determining respective characteristic values that indicate the two or more characteristics of some of the mutants in the library, and scoring the two or more characteristic values as one value per mutant by normalizing and integrating the characteristic values;
3) conducting machine learning by using the score values and ranking the library; and
4) selecting a protein for which two or more characteristics are optimized, based on the ranking results, wherein the two or more characteristic values are numerical values based on different measurement data related to respective different characteristics.

[2] The method according to [1], wherein the two or more characteristic values are relative values or standardized values, for example, values obtained by converting, into numerical values, measurement data related to the characteristics of each mutant as a ratio relative to measurement data each related to characteristics of a target value, for example, a wild-type of the target protein or a protein to be compared (any protein having a "characteristic to be targeted" or "characteristic to be exceeded") or as a standardized value thereof.

[3] The method according to [1] or [2], wherein the scoring is performed according to the following formula (I) :

Score value = $f$(1st characteristic value - reference value of 1st characteristic value) $\times$ $f$(2nd characteristic value - reference value of 2nd characteristic value) ... $\times$ $f$(nth characteristic value - reference value of nth characteristic value)   [Formula I]

wherein $f(x)$ is any selected from the group consisting of a sigmoid function $(x)$, a hyperbolic tangent function $(x)$, a Gaussian function $(x)$, a lognormal distribution function $(x)$, a ReLU function $(x)$, a linear function $(x)$, an n-dimensional function $(x)$, an exponential function $(x)$, a logarithmic function $(x)$, a hyperbolic function $(x)$, and a combination thereof.

[4] The method according to [3], wherein $f(x)$ is a sigmoid function $(x)$, a hyperbolic tangent function $(x)$, a Gaussian function $(x)$, or a lognormal distribution function $(x)$.

[5] The method according to any of [1] to [4], wherein the machine learning is performed by any selected from Bayesian linear regression, Gaussian process regression, decision tree (random forest, gradient boosting decision tree), neural network, deep neural network (convolutional neural network, recurrent neural network, LSTM), k-nearest neighbor algorithm, and support vector machines.

[6] The method according to any of [1] to [5], wherein a site to be mutated is determined by consensus engineering.

[7] The method according to any of [1] to [6], wherein the target protein is an antibody or an enzyme.

[8] A method of producing a protein for which two or more characteristics are optimized, comprising:

1) providing a first library comprising mutants from random mutation of a target protein;
2) determining respective characteristic values that indicate the two or more characteristics of some of the mutants in the first library, and scoring the two or more characteristic values as one value per mutant by normalizing and integrating the characteristic values;
3) conducting machine learning by using the score value and ranking the library; and
4) obtaining a second library that is smaller than the first library, based on the ranking results; and
5) screening the second library to determine a protein for which two or more characteristics are optimized, wherein the two or more characteristic values are based on different measurement data related to respective different characteristics.

[9] A method of producing a library consisting of proteins for which two or more characteristics are optimized, comprising:

1) providing a first library comprising mutants from random mutation of a target protein;
2) determining respective characteristic values that indicate the two or more characteristics of some of the mutants in the first library, and scoring the two or more characteristic values as one value per mutant by normalizing and integrating the characteristic values;
3) conducting machine learning by using the score value and ranking the library; and
4) obtaining a second library that is smaller than the first library, based on the ranking results, wherein the two or more characteristic values are based on different measurement data related to respective different characteristics.

[10] The method according to [8] or [9], wherein the two or more characteristic values are relative values or standardized values, for example, values obtained by converting, into numerical values, measurement data related to the characteristics of each mutant as a ratio relative to measurement data each related to characteristics of a target value, for example, a wild-type of the target protein or a protein to be compared (any protein having a "characteristic to be targeted" or "characteristic to be exceeded") or as a standardized value thereof.

[11] The method according to any of [8] to [10], wherein the scoring is performed according to the following formula (I):

Score value = $f$(1st characteristic value - reference value of 1st characteristic value) $\times$ $f$

(2nd characteristic value - reference value of 2nd characteristic value) ... $\times$ $f$(nth      [Formula (I) ]

characteristic value - reference value of nth characteristic value) wherein $f(x)$ is any selected from the group consisting of a sigmoid function $(x)$, a hyperbolic tangent function $(x)$, a Gaussian function $(x)$, a lognormal distribution function $(x)$, a ReLU function $(x)$, a linear function $(x)$, an n-dimensional function $(x)$, an exponential function $(x)$, a logarithmic function $(x)$, a hyperbolic function $(x)$, and a combination thereof.

[12] The method according to [11], wherein $f(x)$ is a sigmoid function $(x)$, a hyperbolic tangent function $(x)$, a Gaussian function $(x)$, or a lognormal distribution function $(x)$.

[13] The method according to any of [8] to [12], wherein the machine learning is performed by any selected from Bayesian linear regression, linear regression, Gaussian process regression, logistic regression, decision tree, simple perceptron, multilayer perceptron, neural network, deep neural network, k-nearest neighbor algorithm, and support vector machines.

[14] The method according to any of [8] to [13], wherein a site to be mutated is determined by consensus engineering.

[15] The method according to any of [8] to [14], wherein the target protein is an antibody or an enzyme.

[16] A humanized VHH variant, in which amino acid residues at positions 47, 49, 50, and 51 in an amino acid sequence set forth in SEQ ID NO: 3 are any of the following 1) to 5):

1) L (leucine), G (glycine), A (alanine), and S (serine);
2) I (isoleucine), G (glycine), A (alanine), and T (threonine);
3) L (leucine), G (glycine), A (alanine), and T (threonine);
4) V (valine), G (glycine), A (alanine), and S (serine); and
5) I (isoleucine), G (glycine), V (valine), and S (serine), respectively.

Advantageous Effect of Invention

According to the present invention, functions of industrially useful proteins such as antibodies and enzymes are efficiently improved.

Brief Description of Drawings

[Figure 1] Alignment of amino acid sequences of a llama-derived VHH antibody f8c, a human antibody VH, and a humanized VHH antibody hf8c.

[Figure 2] Size-exclusion chromatography of f8c and hf8c purified by IMAC.

[Figure 3] Thermal shift assays of f8c and hf8c purified by IEXC.

[Figure 4] Amino acid residues of which the appearance frequencies at positions 47, and 49 to 51 are 30% or less in the human antibody group, and which are subject to humanization modification (the numbers are according to Chothia numbering).

[Figure 5] Results of plotting the specific expression level and specific binding activity (top) and specific expression level and specific thermostability (bottom) for each of hf8c, and its 4-residue mutants and 1-residue mutants. Dashed lines are the value of hf8c (intersection points are the plots of hf8c).

[Figure 6] Top 20 mutants predicted by machine learning.

[Figure 7] Results of plotting the specific expression level and specific binding activity (top) and specific expression level and specific thermostability (bottom) for top 20 predicted by machine learning. Dashed lines are the value of hf8c (intersection points are the plots of hf8c).

[Figure 8] The conductivity of top 20 predicted by machine learning in case 1, at the elusion point by IEXC.

[Figure 9] Size-exclusion chromatography of f8c and ML-5 purified by IEXC. The column used is Superdex 75 Increase 10/300 GL (manufactured by Cytiva).

[Figure 10] Thermal shift assays of f8c and ML-5 purified by IEXC.

[Figure 11] Target binding assessments by ELISA for f8c and ML-5.

[Figure 12] Correlation results of conductivity of top 20 at the elusion point by IEXC, relative to principal component 1 (PC1) obtained by principal component analysis.

[Figure 13] Correlation plots of principal component 1 (PC1) and principal component 2 (PC2) obtained by principal component analysis of top 20 mutants in cases 1 to 5.

[Figure 14] Amino acid sequences of EGIII and positions to be mutated.

[Figure 15] Results of plotting the specific expression level and specific heat resistance (top) and specific expression level and specific pH tolerance (bottom) for each of a wild-type of EGIII and its 3-residue mutants and 1-residue mutants. Dashed lines are the value of the wild-type (intersection points are the plots of the wild-type) .

[Figure 16] Top 50 mutants predicted by machine learning.

[Figure 17] Results of plotting the specific expression level and specific heat resistance (top) and specific expression level and specific pH tolerance (bottom) for top 50 predicted by machine learning. Dashed lines are the value of the wild-type (intersection points are the plots of the wild-type).

[Figure 18] The rankings of predicted score values of COMBO and LightGBM for the mutants of which the measured values in Figure 17 fall within top 10.

Description of Embodiments

The present invention relates to a method of producing a protein for which two or more characteristics are optimized, and a method of producing a library consisting of proteins for which two or more characteristics are optimized.

Hereinafter, terms and each procedure of the present invention will be described.

1. Production of Initial Library (First Library)

A library comprising mutants from random mutation of a target protein is provided. As used herein, this big library that is provided at the beginning is referred to as "initial library" or "first library" in order to distinguish from a smaller library after concentration, which will be described later. The "initial library" and "first library" are interchangeably used herein.

As the "target protein", it is not particularly limited, and functional proteins that needs characteristic improvement, such as antibodies or enzymes are preferable.

As the site to be mutated ("mutation site"), sites that affect the characteristics to be optimized, preferably sites that affect two or more characteristics to be subject are selected. The phrase "affect the characteristics" means that the characteristics are changed and/or improved due to alteration (substitution, deletion, or insertion) of the amino acid at the corresponding site, especially due to amino acid substitutions.

Selection of mutation sites can be performed by, for example, based on consensus engineering. The "consensus engineering" is a design based on consensus (consensus design or consensus-based engineering), and an approach to enhancing the stability of proteins by modifying protein sequences to make them similar to the consensus sequence obtained from multiple protein alignment of specific family (Porebski and Buckle, "Consensus protein design" Protein Engineering, Design & Selection, 2016, 29(7):245-251, Steipe B., et al., J. Mol. Biol, 1994, 240(3):188-192, and the like).

Specifically, in a case of enzyme functional modification (enzyme thermostability improvement and the like), based on the assumption that the amino acid residues that are frequently selected in nature contribute to the enzyme functional improvement, an amino acid sequence group of the proteins belonging to the same family as the amino acid sequence of the starting protein is subjected to a multiple sequence alignment method (Clustal W, MAFFT or the like) to calculate the appearance frequency of the amino acid sequence at each position of the residues, and then the amino acid residue that is stored at the highest frequency is served as consensus residue. The starting protein is then mutated to a consensus residue at the position of each amino acid residue. On the other hand, for antibodies, based on the assumption that various mutations observed in the germ cell line family result from the elimination of mutations that cause structural instability, the amino acid the most frequently observed at the specific position of the alignment of immunoglobulin (Ig) variable region fragment is considered to be the most favorable amino acid for thermodynamic stability.

By using consensus engineering, functional modification of proteins can be performed with only amino acid sequences without requiring knowledge of the crystal structure or complex in silico calculations. However, if the amino acids without using consensus residues are just substituted to consensus residues, it may often occur that the structural stability is lowered on the contrary, or even if the structural stability improves, another function (e.g., enzymatic activity or antigen-binding activity) is decreased. Therefore, the selection of the position of the corresponding residue and the amino acid to be expressed at that position is important.

For example, in a case of humanized VHH antibodies, which were described later, the stability was lowered by modifying a framework region (FR) of VHH to a framework region of the closest human antibody VH. From this, residues with lower conservation in human antibodies VH are considered to be a cause of destabilization, and therefore, the amino acids with the expression frequencies of 30% or less in the human antibody VH group are selected as sites for contributing to the stabilization of VHH (mutation site).

For mutagenesis, techniques known in the art can be used such as an error-prone PCR method, a random primer method, an overlap extension PCR method, an inverse PCR method, DNA shuffling, a staggered PCR method, a Kunkel method, a quick change method and the like. Commercially available mutagenesis kits can also be used.

The size of the library is not particularly limited and is appropriately determined according to the number of mutation sites. As there are 20 natural amino acids, when the mutation sites are on 3 residues, for example, the size is $20^3$ and about 8,000, when 4 residues, the size is $20^4$ and about 16,000.

2. Assessment and Scoring of Two or More Characteristics

Next, two or more characteristics of a part of mutants in the library are assessed. The number of mutants for which characteristics are assessed is not particularly limited as long as training data meaningful to artificial intelligence can be provided. As the number of the natural amino acids is 20, data of $20 \times n$ or more, preferably $20 \times n + 100$ or more, and more preferably $20 \times n + 100$ to 200 are provided, wherein n is the number of residue positions to be mutated.

The "two or more characteristics" are not particularly limited as long as the characteristics are different characteristics assessed by different measurement data, and examples thereof include biological activity, affinity (binding activity), target specificity, catalytic activity, substrate specificity, structural stability, thermostability, pH stability, aggregability, expression level, salt stability, pressure stability, reduction stability, denaturant stability, solubility, protease resistance, cytotoxicity, enzyme inhibitory activity, antibacterial activity, signal inhibitory activity, and regulatory factor inhibitory activity. Three or more characteristics and/or four or more characteristics can be simultaneously optimized. Combinations of characteristics are not particularly limited.

The characteristics of respective mutants are measured and/or assessed as numerical values (characteristic values) based on different measurement data related to respective characteristics. For example, respective characteristic values of mutants are assessed as relative values to the target value or standardized values as appropriate. Specifically, characteristic values are assessed as a ratio (relative value) relative to the characteristic values that a wild-type of the target protein (target protein before mutagenesis) or a protein to be compared possesses or as a standardized value thereof. The proteins to be compared may be any protein having a "characteristic to be targeted" or "characteristic to be exceeded". For example, in the case of expression level, binding activity, and thermostability, the characteristic values are each assessed as specific expression level, specific binding activity, specific thermostability relative to the expression level, binding activity, and thermostability of a wild-type protein or a protein to be compared, or as standardized expression level, standardized binding activity and standardized thermostability.

In the present invention, the two or more characteristics can be simultaneously optimized by normalizing and integrating the two or more characteristic values, and scoring the values as one value per mutant.

In the present invention, the term "normalization" means to unify the scales of characteristic values that vary in scale (size, unit, or the like). As mentioned above, a plurality of different functions and physical properties based on different measurement data are different in size, unit, and variance, which are often contradictory to each other. When scoring, respective characteristic values are preferably normalized to make their "weight" equal. Methods for normalization are well-known in the art, and are performed by selecting appropriate functions according to the respective characteristic values and distribution thereof. The numerical values after normalization are preferably scaled from 0 to 1 (0 to -1) for every characteristic. The normalized characteristic values are scored as one value by integration.

The scoring is performed according to the following formula, for example.

Score value = $f$(1st characteristic value - reference value of 1st characteristic value) $\times$ $f$(2nd characteristic value - reference value of 2nd characteristic value) ... $\times$ $f$(nth characteristic value - reference value of nth characteristic value)          [Formula I]

As the function "$f(x)$", a sigmoid function $(x),$ a Gaussian function $(x)$ (e.g., normal distribution function), a lognormal distribution function $(x),$ a ReLU function $(x),$ a linear function $(x),$ an n-dimensional function $(x),$ an exponential function $(x),$ a logarithmic function $(x),$ a trigonometric function $(x),$ a hyperbolic function $(x)$ (e.g., hyperbolic tangent function), and a combination thereof can be used.

The term "reference value" means a value that the characteristic value should exceed or the value that should be targeted (minimum required characteristic value or target value).

If it is optimal when the characteristic values are directed to a value greater than the reference value, the reference value is the "minimum required characteristic value". For example, when aiming to a characteristic value higher than that of the wild-type, the characteristic value of the wild-type becomes the reference value. The function for normalization is appropriately selected according to the possible values for the characteristic value. For example, when the rate of change of the characteristic value is maximized near the reference value, the characteristic value can be normalized using the functions such as sigmoid function, exponential function, n-dimensional function (n = odd number), logarithmic function, hyperbolic tangent function or the like. On the other hand, when the characteristic value equal to or lower than the reference value, the characteristic value is constant, and the characteristic value increases when exceeding the reference value, the characteristic value can be normalized using ReLU function or the like.

If it is optimal when the characteristic values are directed to a certain target value, the reference value is the target value. For example, when aiming to obtain a mutant exhibiting X times the characteristics of the wild-type, the characteristic value (target value) that is X times the wild-type becomes the reference value. The function for normalization is appropriately selected according to the possible values for the characteristic value. For example, when the characteristic value exhibits the maximum value near the target value, the characteristic value can be normalized using the functions such as Gaussian function (normal distribution function), lognormal distribution function, n-dimensional function (n = even number), hyperbolic function or the like.

3. Machine Learning by Score Values

Machine learning is performed by using the score values as training data and rank the library. In other words, artificial intelligence is trained on score values obtained for some of the mutants in the library and corresponding sequence information on the mutants to predict scores for all mutants in the library and rank the scores.

Amino acid sequence information is input by converting characters into numerical values (numerical vectors).

Examples of such a method include a method known in the art, and T-scale, Z-scale, ST-scale, BLOSUM, FASGAI, MSWHIM, ProtFP, ProtFP-Characteristic, VHSE, Aromaphilicity, PSSM, and the like can be used (van Westen et al., J Cheminform. 2013; 5: 41).

Machine learning can be performed by Bayesian linear regression, linear regression, Gaussian process regression, logistic regression, decision tree, simple perceptron, multilayer perceptron, neural network, deep neural network, k-nearest neighbor algorithm, and support vector machines. Among others, Bayesian linear regression is preferable.

The decision tree is an algorithm that has a hierarchical tree structure composed of a plurality of conditional branches. Ensemble learning by combining two or more decision trees includes random forest, gradient boosting decision tree, and the like.

The "neural network" is an algorithm to which arbitrary function is applied on activation functions of multilayer perceptron. Examples of such arbitrary functions include a sigmoid function, hyperbolic tangent functions, ReLU function, and the like.

The "deep neural network" is an algorithm having two or more hidden layers of a neural network, and is also referred to as deep learning. Application examples thereof include a convolutional neural network, recurrent neural network, LSTM, GRU, and the like.

The "Gaussian process regression" is a regression approach using a stochastic process on the assumption that the joint probability distribution is Gaussian distribution, and one of the machine learning approaches that determine the optimal values (maximum value or minimum value) of an unknown function (black-box function). However, as the number of dimensions of input increases, the amount of calculation increases exponentially, and therefore, a bigger number of dimensions thereof causes the calculation to be practically difficult. On the other hand, the "Bayesian linear regression" is a regression approach using a linear function, and is a machine learning approach that can determine the optimal values (maximum value or minimum value) of an unknown function (black-box function) same as Gaussian process regression while it can handle a bigger number of dimensions. Each candidate point is represented as a numerical vector called a descriptor. A machine learning model is trained using data of the candidate points previously assessed, and using the trained model, predicted values and predicted variance of the model functions for the remaining candidate points are calculated.

The "Bayesian optimization" is to use a machine learning model constructed by a regression using a Bayesian estimation such as Gaussian process regression and Bayesian linear regression, to calculate scores depending on predicted values and predicted variance, and thereby to set the candidate point with the biggest score as a next assessment point to repeat the function evaluation. The new data obtained here is added to the training data.

For the "Bayesian optimization", known software can be used. Examples thereof are known, such as 2DMAT (https://www.pasums.issp.u-tokyo.ac.jp/2dmat/) COMmon Bayesian Optimization Library (COMBO) (Ueno et al., Mater. Discov., 4, 18-21 (2016), https://tomoki-yamashita.github.io/CrySPY_doc/), CrySPY (https://tomoki-yamashita.github.io/CrySPY_doc/), PHYSBO (optimization tools for PHYsics based on Bayesian Optimization) (https://www.pasums.issp.u-tokyo.ac.jp/physbo/), and the like, but not limited thereto. Among others, COMBO is preferable.

4. Production of Small-Scale Library (Second Library)

By machine learning using data of some of the mutants, artificial intelligence predicts score values obtained for all of the mutants in the library and ranks them. By selecting suitable mutants based on the prediction results, a smaller library than the initial library can be produced. This small library as used herein is referred to as "second library". The "second library" is an enriched library that is composed of mutants more suitable for two or more characteristics.

The library may be enriched two or more times, if necessary. In other words, a second library is produced from the initial library, and then the obtained second library is used as an initial library to produce a third library. By repeating this process, enrichment can be performed as many times as possible. The "two or more characteristics" used for the initial enrichment and those used for the second or subsequent enrichment may be the same or different. At the second or subsequent times, enrichment may be performed for two or more characteristics, or for one characteristic.

5. Determination of Protein for Which Two or More Characteristics are Optimized

By functional prediction through machine learning, mutants for which two or more characteristics are optimized can be selected from the initial library, or the second, third or subsequent libraries. The predicted mutants are actually expressed, and their characteristics are assessed and confirmed to select the best ones. For the consideration of industrial applicability, a smaller number of mutation sites are generally preferable. Therefore, the optimal proteins (mutants) are ultimately determined in consideration of functional improvement and the number of mutations introduced.

6. Optimized Protein

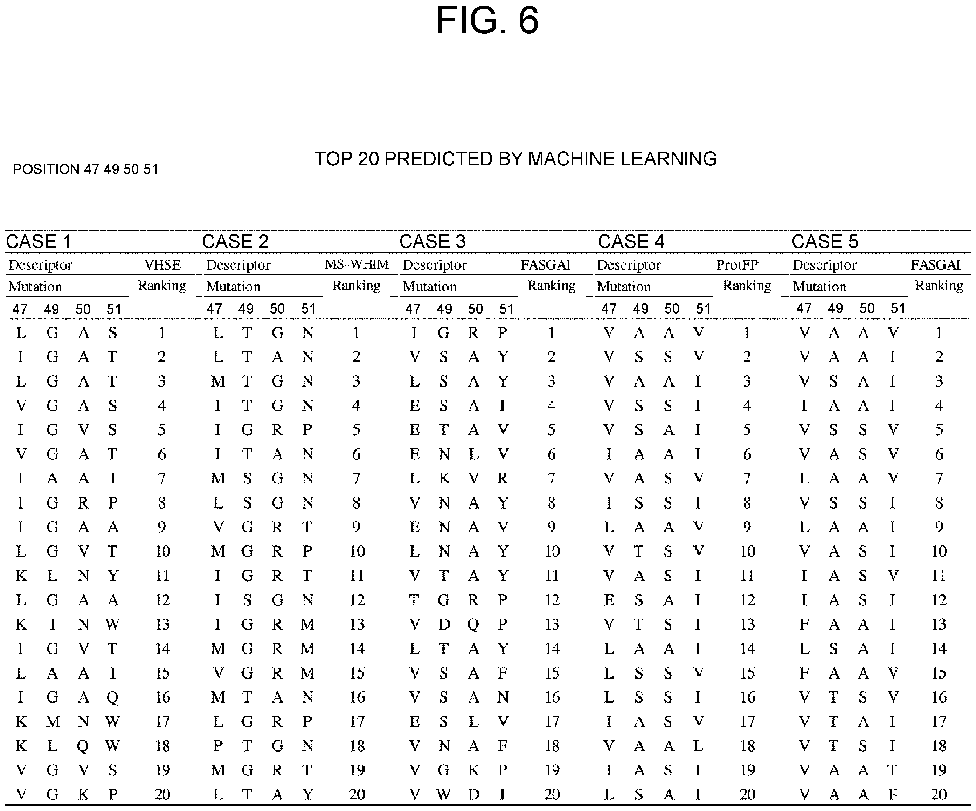

The present invention also provides a protein variant that is optimized by the method of the present invention. For example, humanized VHH variants are provided, in which amino acid residues at positions 47, 49, 50, and 51 in an amino acid sequence set forth in SEQ ID NO: 3 are each substituted to a mutation set at positions 47, 49, 50, and 51 of any of those shown in Figure 6. These VHH variants are superior to conventional known VHHs in expression level, binding activity, and structural stability (thermostability).

Among the humanized VHH variants shown in Figure 6, the amino acid sequences of the VHH variants ranked 1st to 5th (Variants 1 to 5) in case 1 are set forth in SEQ ID NOs: 5 to 9, respectively. Variants 1 to 5 have the following amino acid residues at positions 47, 49, 50, and 51, and have partial substitution from wild-type amino acid residues (I (isoleucine), S (serine), A (alanine), and V (valine), respectively).

Variant 1) L (leucine), G (glycine), A (alanine), and S (serine)
Variant 2) I (isoleucine), G (glycine), A (alanine), and T (threonine)
Variant 3) L (leucine), G (glycine), A (alanine), and T (threonine)
Variant 4) V (valine), G (glycine), A (alanine), and S (serine)
Variant 5) I (isoleucine), G (glycine), V (valine), and S (serine)

* In the order from left, the amino acids at positions 47, 49, 50, and 51 are shown.

Examples

Hereinafter, the present invention will be described in more detail by reference to Examples; however, the present invention is not limited to these Examples.

Example 1: Optimization of Humanized Antibody VHH

Antibodies often have lower structural stability (non-aggregability and heat resistance) of variable region fragments in normal acquisition. In the case of humanization, modification of variable regions may cause target affinity and/or structural stability (non-aggregability and heat resistance) to be lowered. In this example, a llama-derived antibody fragment (VHH antibody) was subjected to simultaneous optimization of the expression level, binding activity, and structural stability.

1.1 Stability of Humanized VHH

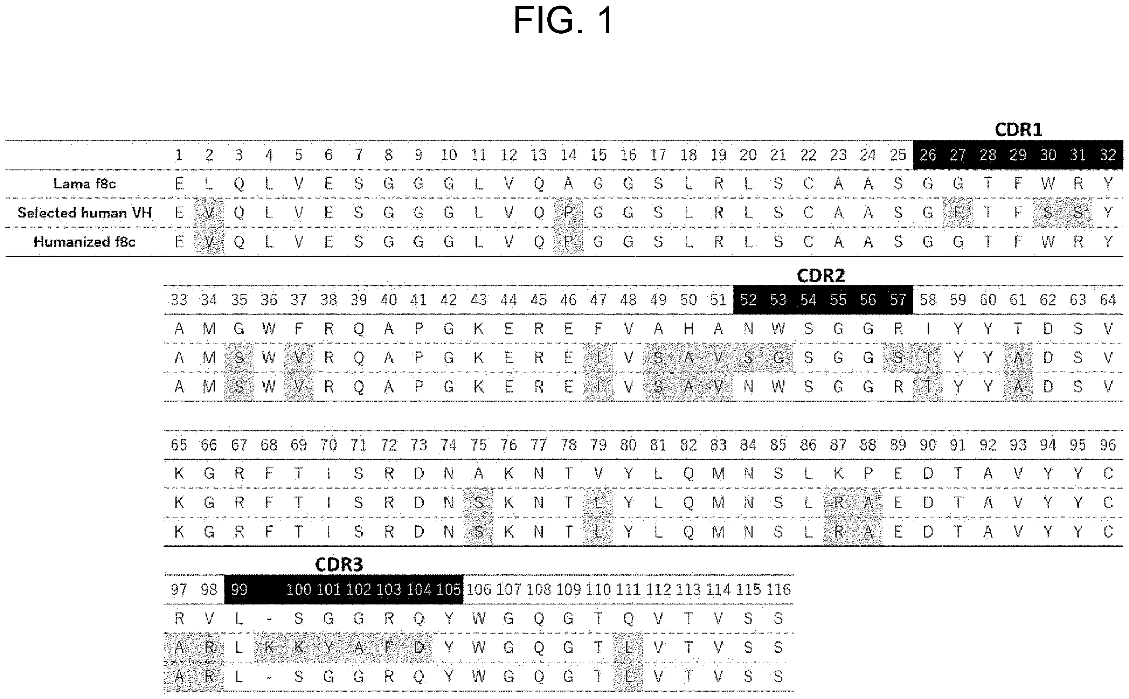

A humanized VHH antibody hf8c (SEQ ID: 3) was produced by converting a framework region of a llama-derived VHH antibody f8c (SEQ ID NO: 1, WO2019/198731) to a human antibody VH (SEQ ID NO: 2) (Figure 1). Escherichia (E.) coli was transformed with respective expression vectors containing f8c and hf8c gene fragments, and cultured in a 2xYT medium containing ampicillin. Bacterial cells were centrifuged to separate into a medium supernatant fraction and bacterial cells, and the bacterial cells were suspended in a phosphate buffer solution and ultrasonically sonicated. After sonication, the cells were centrifuged and a soluble fraction in the bacterial cells were recovered. The fraction was purified with metal ion affinity chromatography (IMAC) (Ni Sepharose 6Fast Flow (manufactured by Cytiva)).

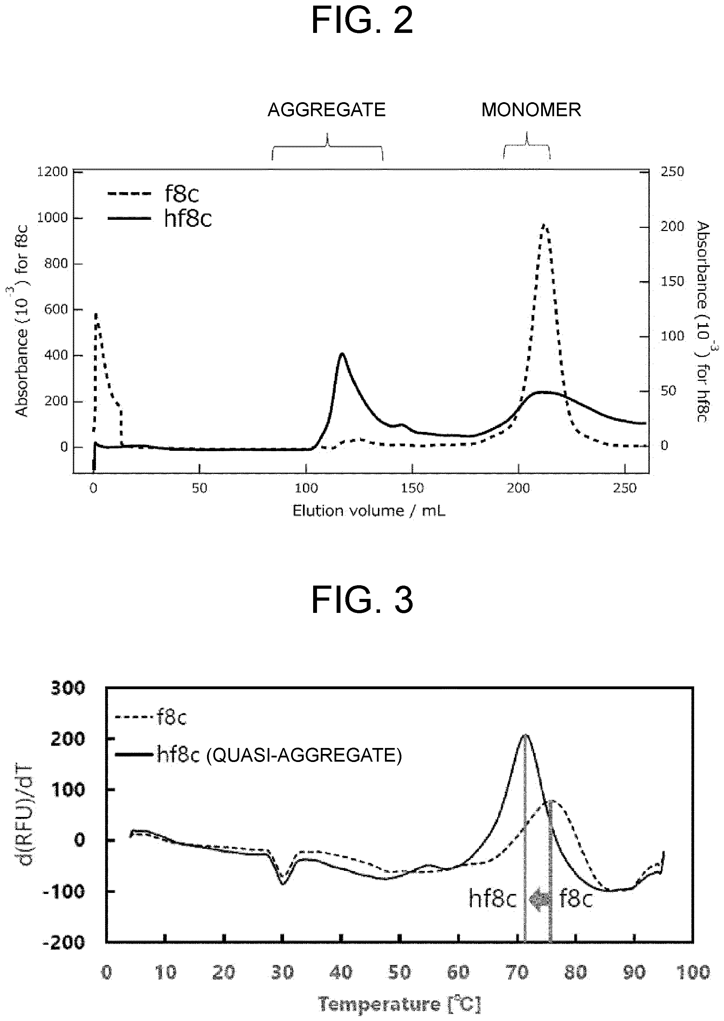

IMAC-purified f8c and hf8c were each subjected to size-exclusion chromatography (SEC) (Superdex 75 26/60 p.g. column (manufactured by Cytiva)). Almost all were aggregated by humanization, the majority thereof were adsorbed on the column, the total amount eluted was small, and most of the eluted part was formed in aggregates and eluted earlier than the fraction originally to be eluted (Figure 2). IMAC-purified f8c and hf8c were also subjected to ion exchange chromatography (IEXC) (RESOURCE S (manufactured by Cytiva)). Compared to f8c, hf8c was eluted at a higher salt concentration (higher conductivity), and its physical properties were confirmed to be changed to those with high column adsorption (aggregation).

A thermal shift assay was performed using IEXC-purified f8c and hf8c to measure the thermostability. It was confirmed that hf8c had a lower thermal denaturation mid-point Tm value than f8c, and the humanization lowered its thermostability (Figure 3).

1.2 Modification of Humanized VHH

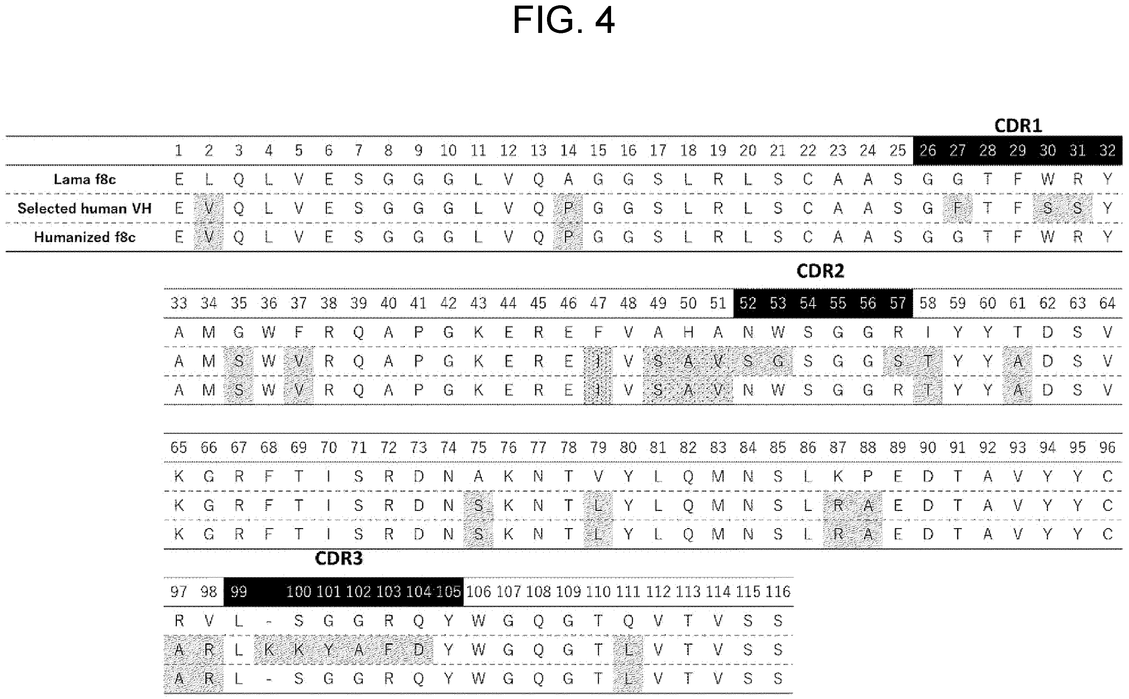

The human antibody VH sequence used for producing hf8c was selected as a human antibody VH sequence with the highest sequence homology to f8c. It was thus considered that the amino acid residues with a lower appearance frequency in humanized sequences may be involved in changes in physical properties of antibodies, and therefore, residues with the appearance frequencies of 30% or less in the human antibody FR regions were investigated (Figure 4). Then, among these residues, 4 residues (47, 49, 50, and 51 in the Figure) which were modified by humanization were set as mutation sites; and separately at each of the target residue positions, 1-residue saturated mutants with substitution to 20 amino acids (1 non-mutant and 19 mutants × 4 = 76 mutants) and simultaneous random mutants (171 mutants) in which all the target residues were simultaneously and randomly mutated were produced.

The expression level, binding activity, and thermostability were assessed with ELISA. Specifically, E. coli transformed with expression vectors each containing a gene fragment of hf8c and mutants with a FLAG tag and a polyhistidine tag at the C-terminus was cultured. The resulting medium supernatant fractions were used to assess the expression level, binding activity, and thermostability by ELISA. The expression level was assessed with values which were obtained by adding a medium supernatant on a plate immobilized with an anti-FLAG antibody and washing, and then measuring the amount of each mutant remained on the plate using an HRP-conjugated anti-polyhistidine tag antibody. The binding activity was assessed with values which were obtained by adding a medium supernatant on a plate immobilized with a Her2 protein, which was a target protein, and washing, and then measuring the amount of each mutant remained on the plate using an HRP-conjugated anti-polyhistidine tag antibody, and standardizing the measured values with the assessed values of the expression level. The thermostability was assessed with values which were obtained by adding a medium supernatant that was heat treated at 54°C for 1 hour, on a plate immobilized with a Her2 protein, measuring the amount of each mutant remained on the plate using an HRP-conjugated anti-polyhistidine tag antibody, and standardizing the measured values with the assessed values of the binding activity.

It was suggested that the expression level as the soluble fraction may be correlated with aggregation suppressing properties (high solubility rate) and thermodynamic structural stability (thermal denaturation mid-point Tm) (Niwa et al., PNAS March 17, 2009 106 (11) 4201-4206, Ito et al., Chemistry Letters, (2021) 50, 1867-187). The expression level may not only be the amount produced as a simple soluble fraction but also be an index of physical properties of aggregation suppressing properties and thermodynamic structural stability (thermal denaturation mid-point Tm).

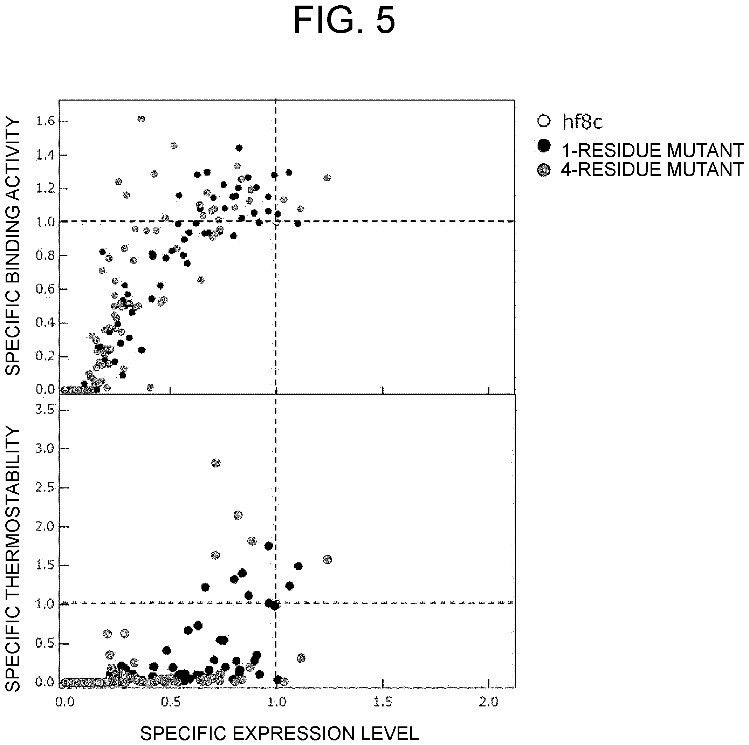

The expression level, binding activity, and thermostability of each mutant were determined as a ratio relative to the assessed values of the expression level, binding activity, and thermostability (specific expression level, specific binding activity, and specific thermostability) of hf8c before mutation, respectively. Plotting the specific expression level and specific binding activity, and specific expression level and specific thermostability revealed that there were a plurality of mutants in which both the binding activity and thermostability were improved compared to hf8c, but there were few mutants in which the expression level was higher than that of hf8c (Figure 5).

1.3 Production of Machine Learning-Based Prediction System

The above data was used as training data, and machine learning was performed in which the functional assessment values of unknown mutants were predicted from the amino acid sequences. A prediction system was produced by Bayesian linear regression using COMBO, which is high-speed Bayesian Optimization software (e.g., Ueno et al., 2016, supra). The sequence data of the mutants were represented by using an adequate index or combination thereof among those expressed as 1 to 10-dimensional vector per residue according to the known reports (van Westen et al., 2013, supra).

The three characteristic values (specific expression level, specific binding activity, and specific thermostability) were scored as one value by the following 5 methods.

Case 1

Score value = $f_1$(specific expression level - 0.8) × $f_2$(specific binding activity - 0.8) × $f_3$(specific thermostability - 1.0)

$$f_1(x) = \text{sigmoid } (x)$$

$$f_2(x) = \text{sigmoid } (x)$$

$$f_3(x) = \text{sigmoid } (x)$$

Case 2

Score value = $f_1$(specific expression level - 0.8) $\times$ $f_2$(specific binding activity - 0.8) $\times$ $f_3$(specific thermostability - 1.0)

$$f_1(x) = \text{sigmoid } (x)$$

$f_2(x)$ = gaussian $(x)$, $\mu$(mean value) = 0.5, $\sigma$ = 0.3 (variance)

$$f_3(x) = \text{sigmoid } (x)$$

Case 3

Score value = $f_1$(specific expression level - 0.8) $\times$ $f_2$(specific binding activity) $\times$ $f_3$(specific thermostability)

$$f_1(x) = \text{sigmoid } (x)$$

$$f_2(x) = \text{gaussian } (x), \mu = 0.5, \sigma = 0.3$$

$$f_3(x) = \text{gaussian } (x), \mu = 2.0, \sigma = 0.6$$

Case 4

Score value = $f_1$(specific expression level - 0.8) $\times$ $f_2$(specific binding activity) $\times$ $f_3$(specific thermostability)

$$f_1(x) = \text{sigmoid } (x)$$

$$f_2(x) = \text{gaussian } (x), \mu = 0.5, \sigma = 0.3$$

$$f_3(x) = \text{gaussian } (x), \mu = 1.5, \sigma = 0.6$$

Case 5

Score value = $f_1$(specific expression level - 0.8) $\times$ $f_2$(specific binding activity) $\times$ $f_3$(specific thermostability)

$$f_1(x) = \text{sigmoid } (x)$$

$$f_2(x) = \text{gaussian } (x), \mu = 0.5, \sigma = 0.3$$

When the specific thermostability is 1.5 or less,

$$f_3(x) = \text{gaussian } (x), \mu = 1.5, \sigma = 0.3$$

When the specific thermostability is more than 1.5,

$$f_3(x) = \text{gaussian } (x), \mu = 1.5, \sigma = 1.0$$

The sigmoid function was used when conducting machine learning so that score values were high when the characteristic values were higher than the reference value. In addition, when an aggregate was formed, ELISA intensity sometimes showed high, and to correct this, a Gaussian function was used when conducting machine learning as a certain value being the optimal value.

In case 1, the sigmoid function was used for all for training to evolve to where the specific expression level, specific binding activity, and specific thermostability become higher than the reference values (multiple number of standardized values when the specific expression level, specific binding activity, and specific thermostability of h8c are 1: 1 $\times$ 0.8 in the case of the specific expression level and specific binding activity; and 1 $\times$ 1 in the case of the specific thermostability).

In case 2, the sigmoid function was used for the specific expression level and specific thermostability, and the Gaussian function was used for the specific binding activity for training to evolve to where the specific expression level and specific thermostability are higher than the reference values, and the optimal value of the specific binding activity is 0.5 times the reference value.

In case 3, the sigmoid function was used for the specific expression level, and the Gaussian function was used for the specific binding activity and specific thermostability for training to evolve to where the specific expression level is higher than the reference value, and the optimal values of the specific binding activity and specific thermostability are 0.5 times and 2 times the reference values, respectively.

In case 4, the sigmoid function was used for the specific expression level, and the Gaussian function was used for the specific binding activity and specific thermostability for training to evolve to where the specific expression level is higher than the reference value, and the optimal values of the specific binding activity and specific thermostability are 0.5 times and 1.5 times the reference values, respectively.

In case 5, as in case 4, the sigmoid function was used for the specific expression level and the Gaussian function was used for the specific binding activity and specific thermostability for training to evolve to where the specific expression level is higher than the reference value, and the optimal values of the specific binding activity and specific thermostability are 0.5 times and 1.5 times the reference values, respectively. However, for the specific thermostability, a Gaussian function in which the standard deviation value is changed around the optimal value was used in order to introduce asymmetry around the set optimal value.

1.4 Selection of Promising Mutant by Prediction System

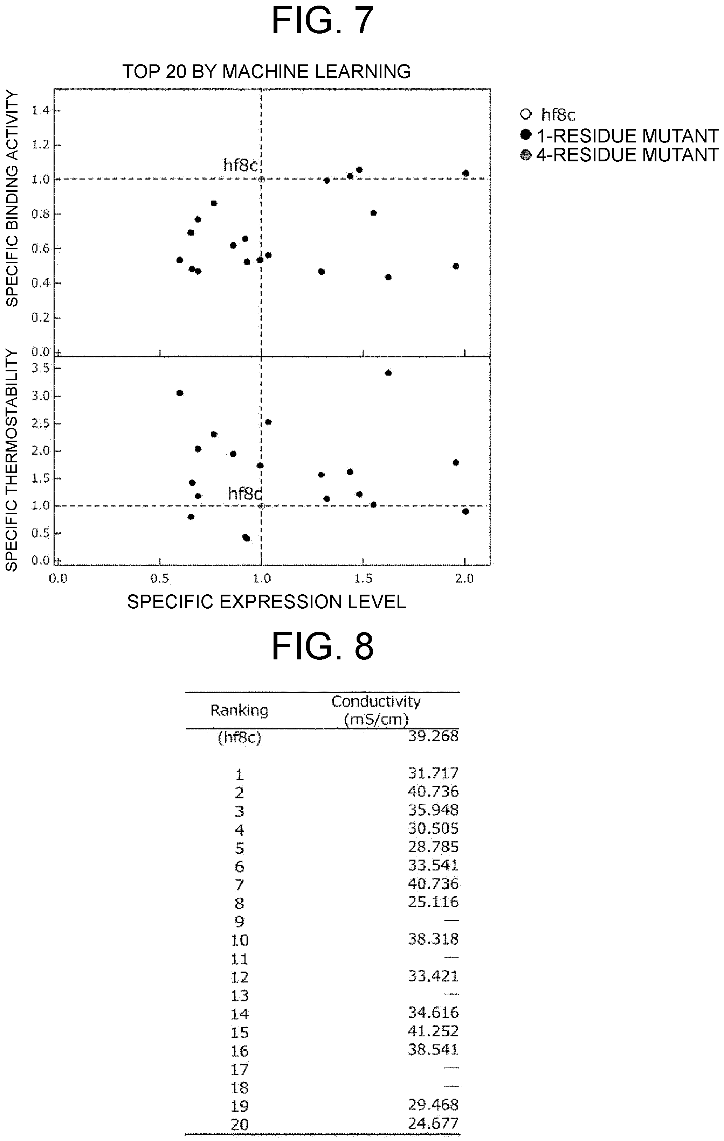

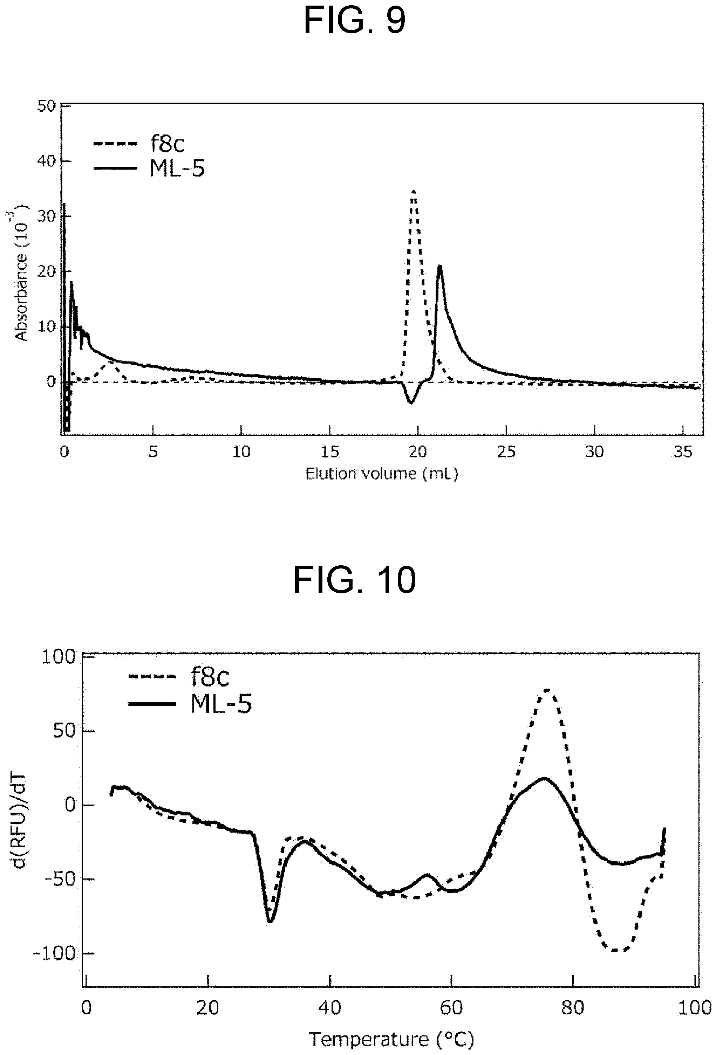

Using the constructed prediction system, predicted score values of all mutants (excluding 247 mutants which were already measured as training data, and hf8c) contained in the sequence space formed by 4 residues (47, 49, 50, and 51 in Figure 4) were calculated (Figure 6: top 20 sequences of the highest score values of each case). Among those, the top 20 mutants of case 1 were expressed in E. coli, samples were purified by IMAC according to 1.1 and 1.2, and the expression level, binding activity, and thermostability were measured by ELISA (Figure 7). As a result, it was possible to obtain mutants of which the expression level was higher and the thermostability was improved than hf8c, which was not observed in the training data. Further, IEXC analysis of the top 20 mutants indicated that most of them were eluted at a lower salt concentration than that of hf8c (lower conductivity), and became mutants with low aggregation properties (Figure 8). Also, among these top 20 mutants, mutants (ML-5) which were monomers, and exhibited the thermostability equivalent to that of f8c before humanization were present (Figures 9, 10, and 11).

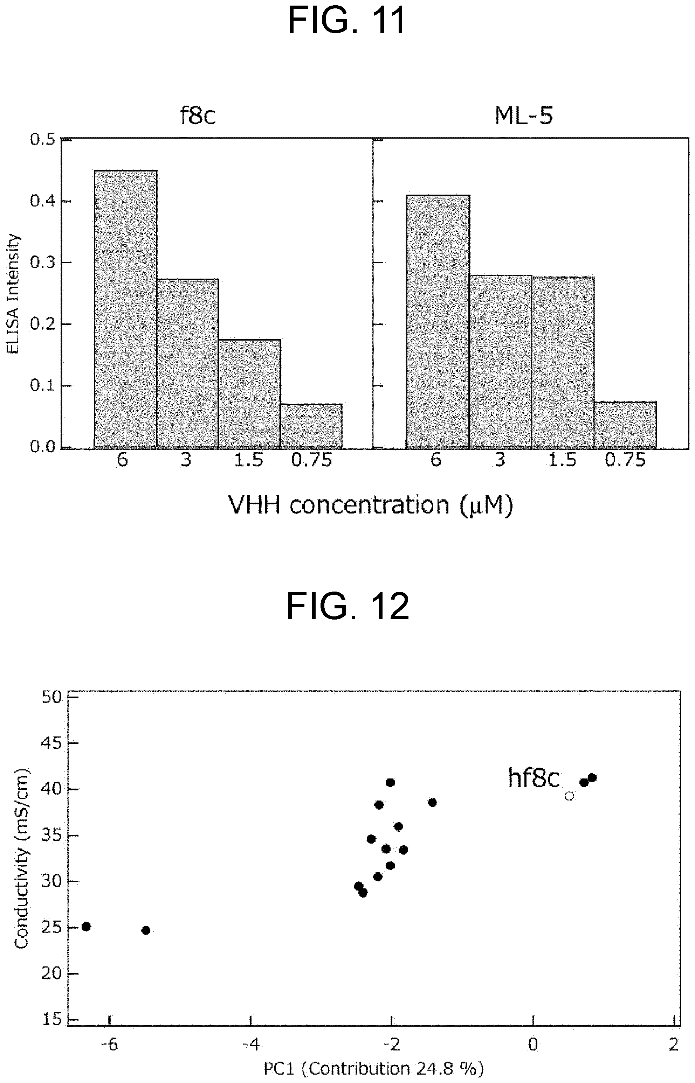

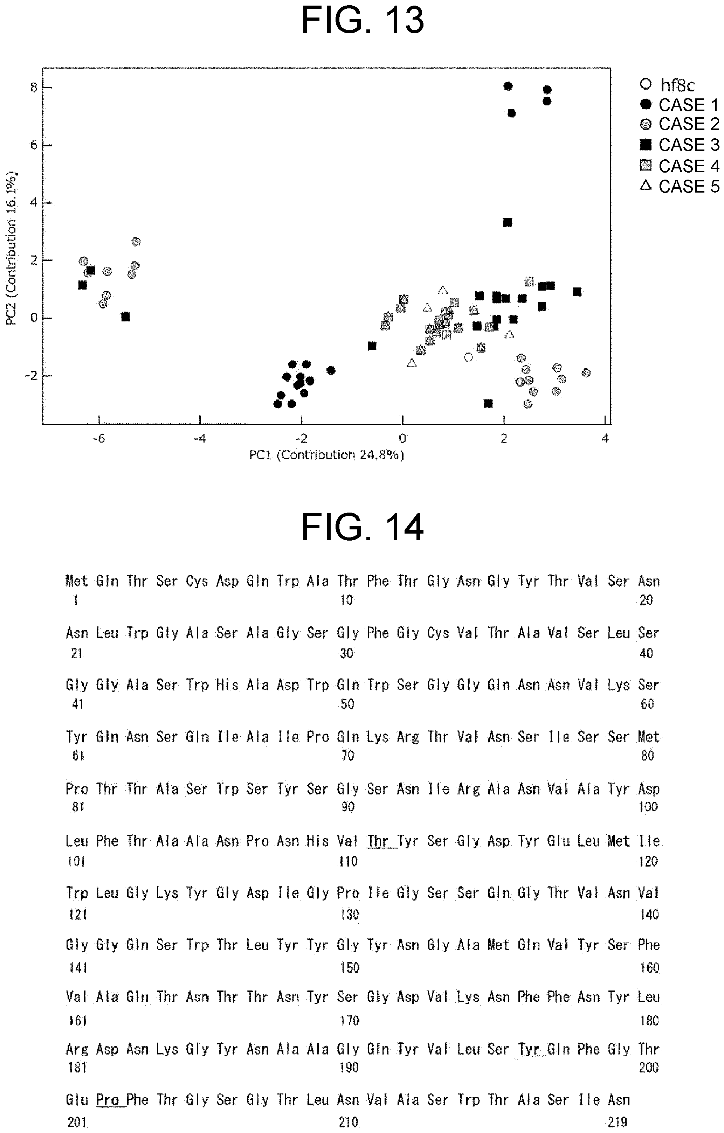

All of the top 20 mutant sequences of cases 1 to 5 were subjected to principal component analysis using the characteristic amount VHSE. As a result, the conductivity in which the sequence of case 1 was eluted by IEXC had a positive correlation with the principal component (PC1) with the highest contribution rate obtained by the principal component analysis, and a component index showing an aggregation suppressing effect was able to be derived (Figure 12). Comparing to PC1 of each case to each other, in cases 2 and 3, there were mutants showing lower PC1 values than those of mutants of case 1, suggesting that these were mutants showing a better aggregation suppressing effect (Figure 13).

Example 2: Optimization of Enzyme

Using enzymes improves the thermostability and pH stability to increase an enzymatic reaction temperature and make enzymes reusable, whereby reducing costs is expected. In this example, a Trichoderma reesei-derived cellulase (EGIII, Cel12A, SEQ ID NO: 4) was subjected to simultaneous optimization of the expression level, thermostability, and pH stability.

2.1 Modification of EGIII

Evolutionary engineering modification of EGIII was previously conducted using error-prone PCR, and T111, Y196 and P202 were identified as the positions of the amino acid residues relating to the expression level, thermostability, and pH stability (Figure 14, Nakazawa et al., Appl Microbiol Biotechnol. 2009; 83(4):649-57). In order to find a further optimal combination of mutants for these 3 amino acid residues, separately at each of the target residue positions 111, 196, and 202 of the amino acid residues, 1-residue saturated mutants with substitution to 20 amino acids (1 non-mutant and 19 mutants $\times$ 3 = 58 mutants) and simultaneous random mutants (203 mutants) in which all the target residues were simultaneously and randomly mutated were produced.

First, the expression level, thermostability, and pH stability of the produced 3-residue simultaneous random enzymes were assessed using the enzyme activity as an index. E. coli BL 21 strain which was transformed with expression vectors having each mutant gene was microcultured with 1 mM IPTG induction, 1 mL of cultured bacterial cells were collected, and 200 $\mu$L of 20 mM acetate buffer solution (pH 5.0) was added and ultrasonically sonicated to assess each characteristic using 40 $\mu$L of the supernatant obtained after centrifuge.

The thermostability was assessed by the following procedure: the supernatant at pH 5.0 was warmed at 60°C for 30 minutes; then 15 $\mu$L of 1 M acetate buffer solution was added thereto; carboxymethylcellulose at a final concentration of 1% was used as a substrate to conduct enzymatic reaction at 50°C for 3 hours; and then the amount of generated reducing sugar was measured with TZ assay. The pH stability was assessed by the following procedure: borate buffer solution at pH 9.0 was warmed at 50°C for 30 minutes; then 15 $\mu$L of 1 M acetate buffer solution was added thereto to set back to pH 5.0; and then, carboxymethylcellulose at a final concentration of 1% was used as a substrate to conduct enzymatic reaction at 50°C for 3 hours. The expression level was calculated from the enzymatic reaction at pH 5.0 and 50°C. The 1-residue saturated mutant was similarly assessed.

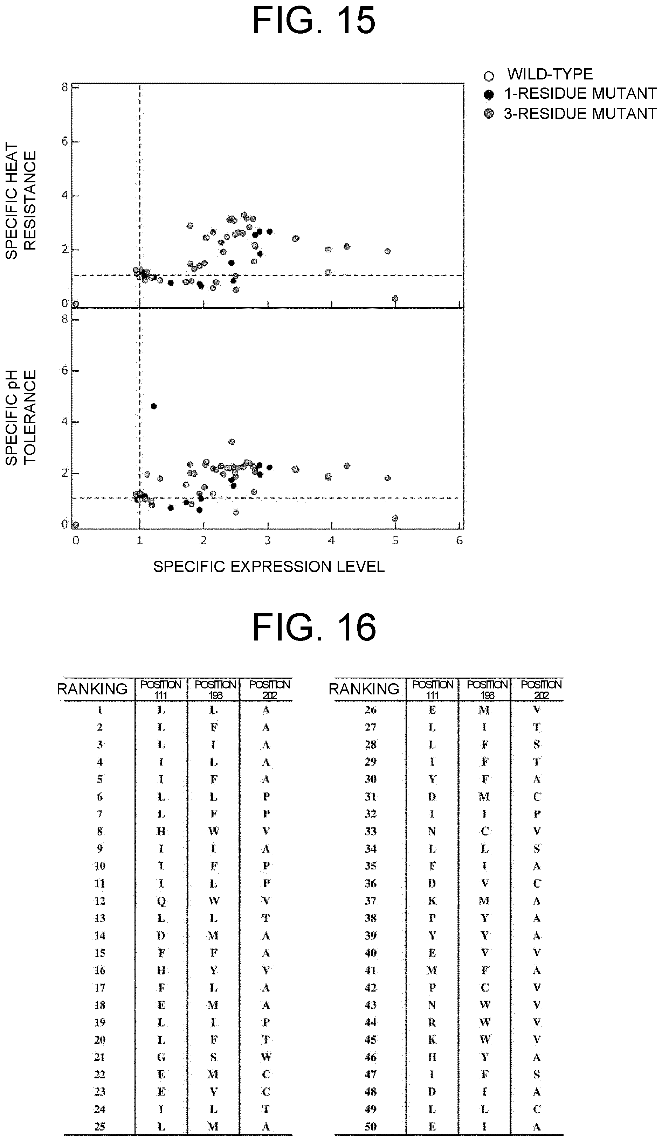

The expression level, heat resistance, and pH tolerance of each mutant were determined as a ratio relative to the expression level, heat resistance, and pH tolerance (specific expression level, specific heat resistance, and specific pH tolerance) of the wild-type EGIII before mutation, respectively. Plotting the specific expression level and specific binding activity, and specific expression level and specific thermostability revealed that there were a plurality of mutants in which both the binding activity and thermostability were improved compared to the wild-type EGIII (Figure 15). When the specific expression level was 0.9 or less, the expression level was assessed as 0. However, 15 mutants with a specific expression level of 0.9 or less for 1-residue mutant were not used as data.

2.2 Production of Machine Learning-Based Prediction System

The above data was used as training data, and machine learning was performed in which the functional assessment values of unknown mutants were predicted from the amino acid sequences. A prediction system was produced using COMBO, which is high-speed Bayesian Optimization software (e.g., Ueno et al., 2016, supra). The sequence data of 3-residue mutants and 1-residue mutants (excluded when no activity found) were used. The sequence data of the mutants were represented by using an adequate index or combination thereof among those expressed as 1 to 10-dimensional vector per residue according to the known reports (Tian F., et al., 2007, supra).

The three characteristic values (expression level, heat resistance, and pH tolerance) were scored as one value by the following formula.

Score value = *f*(specific expression level - 1) × *f*(specific heat resistance - 1) × *f*(specific pH tolerance - 1)

$$f(x) = \mathrm{sigmoid}\ (x)$$

2.3 Selection of Promising Mutant by Prediction System

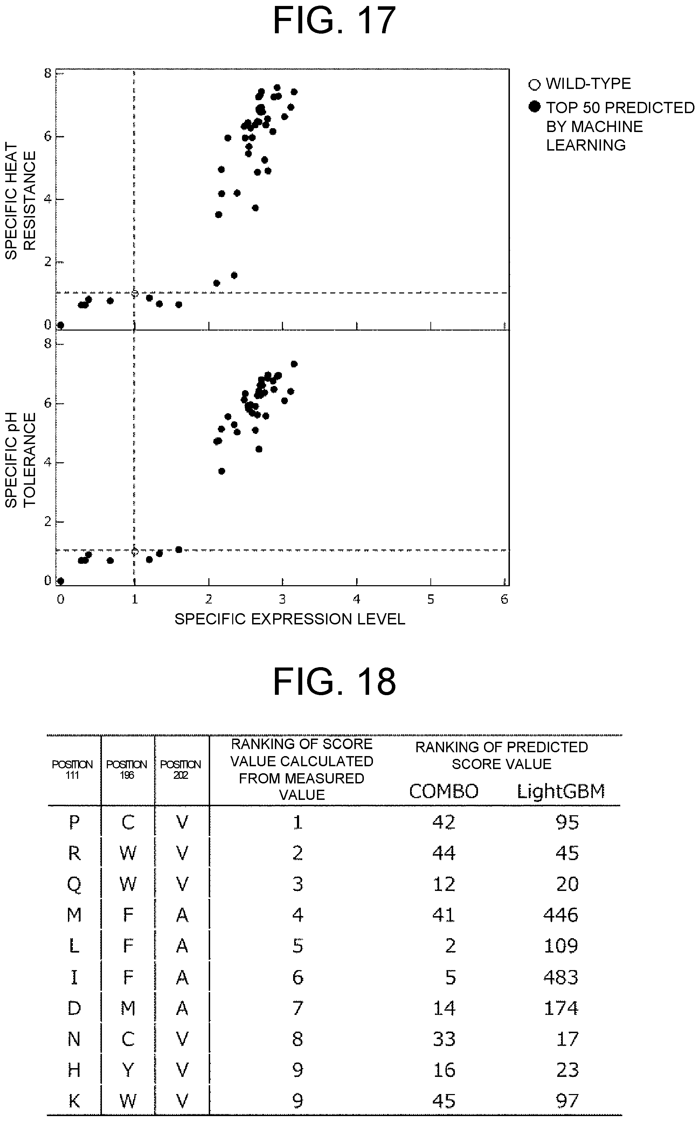

Using the constructed prediction system, predicted score values of all mutants (excluding 261 mutants which were already measured as training data, and wild-type) contained in the sequence space formed by 3 residues (T111, Y196, and P202) were calculated (Figure 16: top 50 sequences of the highest score values). Then, the top 50 mutants were expressed in E. coli, samples were prepared according to 2.2, and the expression level, thermostability, and pH tolerance were measured (Figure 17). As a result, it was found that many mutants had simultaneously improved expression level, thermostability, and pH tolerance compared to the training data.

Further, a regression method different from Bayesian linear regression was conducted. The same training data and score values as those described in 2.2 were used, the gradient boosting decision tree software, LightGBM (https://github.com/microsoft/LightGBM/releases/tag/v3.3. 2) was used to produce a prediction system by a regression method for decision tree, and predicted score values of unmeasured mutants contained in the sequence space formed by 3 residues (T111, Y196, and P202) were calculated. For the top 10 mutants of the highest score values calculated from the measured values among the mutants actually measured in Figure 17, the ranking positions within the predicted score values were examined (Figure 18). As a result, it was found that 6 out of the top 10 mutants of the highest measured values could be obtained when creating the predicted rankings up to top 100 in the decision tree; and although the acquisition rate of high performance mutants was lower than that from Bayesian linear regression by COMBO, high performance mutants can be obtained by another regression.

Industrial Applicability

According to the present invention, it is possible to obtain amino acid sequence information for proteins with high industrial applicability, such as antibodies and enzymes for which two or more characteristics are optimized simultaneously. Accordingly, modification of the proteins for the purpose of functional improvement can be easily performed.

All publications, patents and patent applications cited in this specification shall be incorporated herein by reference.

[Sequence Listing]

SEQ ID NO: 3: Humanized VHH (clone f8c)
SEQ ID NO: 5: Humanized VHH valiant 1
SEQ ID NO: 6: Humanized VHH valiant 2
SEQ ID NO: 7: Humanized VHH valiant 3
SEQ ID NO: 8: Humanized VHH valiant 4
SEQ ID NO: 9: Humanized VHH valiant 5

**Claims**

**1.** A method of producing a protein for which two or more characteristics are optimized, comprising:

1) providing a library comprising mutants from random mutation of a target protein;
2) determining respective characteristic values that indicate the two or more characteristics of some of the mutants in the library, and scoring the two or more characteristic values as one value per mutant by normalizing and integrating the characteristic values;
3) conducting machine learning by using the score values and ranking the library; and
4) selecting a protein for which two or more characteristics are optimized, based on the ranking results, wherein the two or more characteristic values are numerical values based on different measurement data related to respective different characteristics.

**2.** The method according to claim 1, wherein the two or more characteristic values are values each obtained by converting, into numerical values, measurement data related to the characteristics of each mutant as a ratio to a target value.

**3.** The method according to claim 1, wherein the scoring is performed according to the following formula (I):

Score value = $f$(1st characteristic value - reference value of 1st characteristic value) $\times$ $f$(2nd characteristic value - reference value of 2nd characteristic value) ... $\times$ $f$(nth characteristic value - reference value of nth characteristic value)   [Formula I]

wherein $f(x)$ is any selected from the group consisting of a sigmoid function $(x)$, a hyperbolic tangent function $(x)$, a Gaussian function $(x)$, a lognormal distribution function $(x)$, a ReLU function $(x)$, a linear function $(x)$, an n-dimensional function $(x)$, an exponential function $(x)$, a logarithmic function $(x)$, a hyperbolic function $(x)$, and a combination thereof.

**4.** The method according to claim 3, wherein $f(x)$ is a sigmoid function $(x)$, a hyperbolic tangent function $(x)$, a Gaussian function $(x)$, or a lognormal distribution function $(x)$.

**5.** The method according to claim 1, wherein the machine learning is performed by any selected from Bayesian linear regression, linear regression, Gaussian process regression, logistic regression, decision tree, simple perceptron, multilayer perceptron, neural network, deep neural network, k-nearest neighbor algorithm, and support vector machines.

**6.** The method according to claim 1, wherein a site to be mutated is determined by consensus engineering.

**7.** The method according to any of claims 1 to 6,
wherein the target protein is an antibody or an enzyme.

**8.** A method of producing a protein for which two or more characteristics are optimized, comprising:

1) providing a first library comprising mutants from random mutation of a target protein;
2) determining respective characteristic values that indicate the two or more characteristics of some of the mutants in the first library, and scoring the two or more characteristic values as one value per mutant by normalizing and integrating the characteristic values;
3) conducting machine learning by using the score value and ranking the library;
4) obtaining a second library that is smaller than the first library, based on the ranking results; and
5) screening the second library to determine a protein for which two or more characteristics are optimized, wherein the two or more characteristic values are based on different measurement data related to respective different characteristics.

**9.** A method of producing a library consisting of proteins for which two or more characteristics are optimized, comprising:

1) providing a first library comprising mutants from random mutation of a target protein;
2) determining respective characteristic values that indicate the two or more characteristics of some of the mutants in the first library, and scoring the two or more characteristic values as one value per mutant by normalizing and integrating the characteristic values;
3) conducting machine learning by using the score value and ranking the library; and
4) obtaining a second library that is smaller than the first library, based on the ranking results, wherein the two or more characteristic values are based on different measurement data related to respective different characteristics.

**10.** The method according to claim 8 or 9, wherein the two or more characteristic values are values each obtained by converting, into numerical values, measurement data related to the characteristics of each mutant as a ratio to a target value.

**11.** The method according to claim 8 or 9, wherein the scoring is performed according to the following formula (I) :

Score value = $f$(1st characteristic value - reference value of 1st characteristic value) $\times$ $f$ (2nd characteristic value - reference value of 2nd characteristic value) ... $\times$ $f$(nth characteristic value - reference value of nth characteristic value)                    [Formula (I) ]

wherein $f(x)$ is any selected from the group consisting of a sigmoid function $(x)$, a hyperbolic tangent function $(x)$, a Gaussian function $(x)$, a lognormal distribution function $(x)$, a ReLU function $(x)$, a linear function $(x)$, an n-dimensional function $(x)$, an exponential function $(x)$, a logarithmic function $(x)$, a hyperbolic function $(x)$, and a combination thereof.

12. The method according to claim 11, wherein $f(x)$ is a sigmoid function $(x)$, a hyperbolic tangent function $(x)$, a Gaussian function $(x)$, or a lognormal distribution function $(x)$.

13. The method according to claim 8 or 9, wherein the machine learning is performed by any selected from Bayesian linear regression, linear regression, Gaussian process regression, logistic regression, decision tree, simple perceptron, multilayer perceptron, neural network, deep neural network, k-nearest neighbor algorithm, and support vector machines.

14. The method according to claim 8 or 9, wherein a site to be mutated is determined by consensus engineering.

15. The method according to claim 8 or 9, wherein the target protein is an antibody or an enzyme.

16. A humanized VHH variant, wherein amino acid residues at positions 47, 49, 50, and 51 in an amino acid sequence set forth in SEQ ID NO: 3 are any of the following 1) to 5) :

1) L (leucine), G (glycine), A (alanine), and S (serine);
2) I (isoleucine), G (glycine), A (alanine), and T (threonine);
3) L (leucine), G (glycine), A (alanine), and T (threonine);
4) V (valine), G (glycine), A (alanine), and S (serine); and
5) I (isoleucine), G (glycine), V (valine), and S (serine), respectively.

# FIG. 1

**CDR1**

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|---|---|---|---|---|---|---|---|---|---|---|---|---|---|---|---|
| Lama f8c | E | L | Q | L | V | E | S | G | G | G | L | V | Q | A | G | G | S | L | R | L | S | C | A | A | S | G | G | T | F | W | R | Y |
| Selected human VH | E | V | Q | L | V | E | S | G | G | G | L | V | Q | P | G | G | S | L | R | L | S | C | A | A | S | G | F | T | F | S | S | Y |
| Humanized f8c | E | V | Q | L | V | E | S | G | G | G | L | V | Q | P | G | G | S | L | R | L | S | C | A | A | S | G | G | T | F | W | R | Y |

**CDR2**

| 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|---|---|---|---|---|---|---|---|---|---|---|---|---|---|---|
| A | M | G | W | F | R | Q | A | P | G | K | E | R | E | F | V | A | H | A | N | W | S | G | G | R | I | Y | Y | T | D | S | V |
| A | M | S | W | V | R | Q | A | P | G | K | E | R | E | I | V | S | A | V | S | G | S | G | G | S | T | Y | Y | A | D | S | V |
| A | M | S | W | V | R | Q | A | P | G | K | E | R | E | I | V | S | A | V | N | W | S | G | G | R | T | Y | Y | A | D | S | V |

| 65 | 66 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|---|---|---|---|---|---|---|---|---|---|---|---|---|---|---|
| K | G | R | F | T | I | S | R | D | N | A | K | N | T | V | Y | L | Q | M | N | S | L | K | P | E | D | T | A | V | Y | Y | C |
| K | G | R | F | T | I | S | R | D | N | S | K | N | T | L | Y | L | Q | M | N | S | L | R | A | E | D | T | A | V | Y | Y | C |
| K | G | R | F | T | I | S | R | D | N | S | K | N | T | L | Y | L | Q | M | N | S | L | R | A | E | D | T | A | V | Y | Y | C |

**CDR3**

| 97 | 98 | 99 | 100 | 101 | 102 | 103 | 104 | 105 | 106 | 107 | 108 | 109 | 110 | 111 | 112 | 113 | 114 | 115 | 116 |
|---|---|---|---|---|---|---|---|---|---|---|---|---|---|---|---|---|---|---|---|
| R | V | L | - | S | G | G | R | Q | Y | W | G | Q | G | T | Q | V | T | V | S | S |
| A | R | L | K | K | Y | A | F | D | Y | W | G | Q | G | T | L | V | T | V | S | S |
| A | R | L | - | S | G | G | R | Q | Y | W | G | Q | G | T | L | V | T | V | S | S |

# FIG. 2

# FIG. 3

# FIG. 4

|  | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|---|---|---|---|---|---|---|---|---|---|---|---|---|---|---|---|
| | | | | | | | | | | | | | | | | | | | | | | | | | | CDR1 | | | | | | |
| Lama f8c | E | L | Q | L | V | E | S | G | G | G | L | V | Q | A | G | G | S | L | R | L | S | C | A | A | S | G | G | T | F | W | R | Y |
| Selected human VH | E | V | Q | L | V | E | S | G | G | G | L | V | Q | P | G | G | S | L | R | L | S | C | A | A | S | G | F | T | F | S | S | Y |
| Humanized f8c | E | V | Q | L | V | E | S | G | G | G | L | V | Q | P | G | G | S | L | R | L | S | C | A | A | S | G | F | T | F | W | R | Y |

|  | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|---|---|---|---|---|---|---|---|---|---|---|---|---|---|---|---|
| | | | | | | | | | | | | | | | | | | | | CDR2 | | | | | | | | | | | | |
| | A | M | G | W | F | R | Q | A | P | G | K | E | R | E | F | V | A | H | A | N | W | S | G | G | R | I | Y | Y | T | D | S | V |
| | A | M | S | W | V | R | Q | A | P | G | K | E | R | E | F | V | S | I | V | S | A | V | S | G | S | T | Y | Y | A | D | S | V |
| | A | M | S | W | V | R | Q | A | P | G | K | E | R | E | F | V | S | I | V | S | A | V | N | W | S | G | R | L | Y | Y | A | D | S | V |

|  | 65 | 66 | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|---|---|---|---|---|---|---|---|---|---|---|---|---|---|---|---|
| | K | G | R | F | T | I | S | R | D | N | A | K | N | T | V | Y | L | Q | M | N | S | L | K | P | E | D | T | A | V | Y | Y | C |
| | K | G | R | F | T | I | S | R | D | N | S | K | N | T | L | Y | L | Q | M | N | S | L | R | A | E | D | T | A | V | Y | Y | C |
| | K | G | R | F | T | I | S | R | D | N | S | K | N | T | L | Y | L | Q | M | N | S | L | R | A | E | D | T | A | V | Y | Y | C |

|  | 97 | 98 | 99 | 100 | 101 | 102 | 103 | 104 | 105 | 106 | 107 | 108 | 109 | 110 | 111 | 112 | 113 | 114 | 115 | 116 |
|---|---|---|---|---|---|---|---|---|---|---|---|---|---|---|---|---|---|---|---|---|
| | | | CDR3 | | | | | | | | | | | | | | | | | |
| | R | V | L | - | S | G | G | R | Q | Y | W | G | Q | G | T | Q | V | T | V | S | S |
| | A | R | L | K | K | Y | A | F | D | Y | W | G | Q | G | T | L | V | T | V | S | S |
| | A | R | L | - | S | G | G | R | Q | Y | W | G | Q | G | T | L | V | T | V | S | S |

# FIG. 5

# FIG. 6

TOP 20 PREDICTED BY MACHINE LEARNING

POSITION 47 49 50 51

| CASE 1 | | | | | CASE 2 | | | | | CASE 3 | | | | | CASE 4 | | | | | CASE 5 | | | | |
|---|---|---|---|---|---|---|---|---|---|---|---|---|---|---|---|---|---|---|---|---|---|---|---|---|---|
| Descriptor | | | | VHSE | Descriptor | | | | MS-WHIM | Descriptor | | | | FASGAI | Descriptor | | | | ProtFP | Descriptor | | | | FASGAI |
| Mutation | | | | Ranking | Mutation | | | | Ranking | Mutation | | | | Ranking | Mutation | | | | Ranking | Mutation | | | | Ranking |
| 47 | 49 | 50 | 51 | | 47 | 49 | 50 | 51 | | 47 | 49 | 50 | 51 | | 47 | 49 | 50 | 51 | | 47 | 49 | 50 | 51 | |
| L | G | A | S | 1 | L | T | G | N | 1 | I | G | R | P | 1 | V | A | A | V | 1 | V | A | A | V | 1 |
| I | G | A | T | 2 | L | T | A | N | 2 | V | S | A | Y | 2 | V | S | S | V | 2 | V | A | A | I | 2 |
| L | G | A | T | 3 | M | T | G | N | 3 | L | S | A | Y | 3 | V | A | A | I | 3 | V | S | A | I | 3 |
| V | G | A | S | 4 | I | T | G | N | 4 | E | S | A | I | 4 | V | S | S | I | 4 | I | A | A | I | 4 |
| I | G | V | S | 5 | I | G | R | P | 5 | E | T | A | V | 5 | V | S | A | I | 5 | V | S | S | V | 5 |
| V | G | A | T | 6 | I | T | A | N | 6 | E | N | L | V | 6 | I | A | A | I | 6 | V | A | S | V | 6 |
| I | A | A | I | 7 | M | S | G | N | 7 | L | K | V | R | 7 | V | A | S | V | 7 | L | A | A | V | 7 |
| I | G | R | P | 8 | L | S | G | N | 8 | V | N | A | Y | 8 | I | S | S | I | 8 | V | S | S | I | 8 |
| I | G | A | A | 9 | V | G | R | T | 9 | E | N | A | V | 9 | L | A | A | V | 9 | L | A | A | I | 9 |
| L | G | V | T | 10 | M | G | R | P | 10 | L | N | A | Y | 10 | V | T | S | V | 10 | V | A | S | I | 10 |
| K | L | N | Y | 11 | I | G | R | T | 11 | V | T | A | Y | 11 | V | A | S | I | 11 | I | A | S | V | 11 |
| L | G | A | A | 12 | I | S | G | N | 12 | T | G | R | P | 12 | E | S | A | I | 12 | I | A | S | I | 12 |
| K | I | N | W | 13 | I | G | R | M | 13 | V | D | Q | P | 13 | V | T | S | I | 13 | F | A | A | I | 13 |
| I | G | V | T | 14 | M | G | R | M | 14 | L | T | A | Y | 14 | L | A | A | I | 14 | L | S | A | I | 14 |
| L | A | A | I | 15 | V | G | R | M | 15 | V | S | A | F | 15 | L | S | S | V | 15 | F | A | A | V | 15 |
| I | G | A | Q | 16 | M | T | A | N | 16 | V | S | A | N | 16 | L | S | S | I | 16 | V | T | S | V | 16 |
| K | M | N | W | 17 | L | G | R | P | 17 | E | S | L | V | 17 | I | A | S | V | 17 | V | T | A | I | 17 |
| K | L | Q | W | 18 | P | T | G | N | 18 | V | N | A | F | 18 | V | A | A | L | 18 | V | T | S | I | 18 |
| V | G | V | S | 19 | M | G | R | T | 19 | V | G | K | P | 19 | I | A | S | I | 19 | V | A | A | T | 19 |
| V | G | K | P | 20 | L | T | A | Y | 20 | V | W | D | I | 20 | L | S | A | I | 20 | V | A | A | F | 20 |

# FIG. 7

TOP 20 BY MACHINE LEARNING

O  hf8c
● 1-RESIDUE MUTANT
◉ 4-RESIDUE MUTANT

# FIG. 8

| Ranking | Conductivity (mS/cm) |
|---|---|
| (hf8c) | 39.268 |
| 1 | 31.717 |
| 2 | 40.736 |
| 3 | 35.948 |
| 4 | 30.505 |
| 5 | 28.785 |
| 6 | 33.541 |
| 7 | 40.736 |
| 8 | 25.116 |
| 9 | — |
| 10 | 38.318 |
| 11 | — |
| 12 | 33.421 |
| 13 | — |
| 14 | 34.616 |
| 15 | 41.252 |
| 16 | 38.541 |
| 17 | — |
| 18 | — |
| 19 | 29.468 |
| 20 | 24.677 |

# FIG. 9

# FIG. 10

# FIG. 11

f8c          ML-5

ELISA Intensity

VHH concentration (µM)

# FIG. 12

Conductivity (mS/cm)

hf8c

PC1 (Contribution 24.8 %)

# FIG. 13

# FIG. 14

```
Met Gln Thr Ser Cys Asp Gln Trp Ala Thr Phe Thr Gly Asn Gly Tyr Thr Val Ser Asn
1                             10                                          20

Asn Leu Trp Gly Ala Ser Ala Gly Ser Gly Phe Gly Cys Val Thr Ala Val Ser Leu Ser
21                            30                                          40

Gly Gly Ala Ser Trp His Ala Asp Trp Gln Trp Ser Gly Gly Gln Asn Asn Val Lys Ser
41                            50                                          60

Tyr Gln Asn Ser Gln Ile Ala Ile Pro Gln Lys Arg Thr Val Asn Ser Ile Ser Ser Met
61                            70                                          80

Pro Thr Thr Ala Ser Trp Ser Tyr Ser Gly Ser Asn Ile Arg Ala Asn Val Ala Tyr Asp
81                            90                                         100

Leu Phe Thr Ala Ala Asn Pro Asn His Val Thr Tyr Ser Gly Asp Tyr Glu Leu Met Ile
101                           110                                        120

Trp Leu Gly Lys Tyr Gly Asp Ile Gly Pro Ile Gly Ser Ser Gln Gly Thr Val Asn Val
121                           130                                        140

Gly Gly Gln Ser Trp Thr Leu Tyr Tyr Gly Tyr Asn Gly Ala Met Gln Val Tyr Ser Phe
141                           150                                        160

Val Ala Gln Thr Asn Thr Thr Asn Tyr Ser Gly Asp Val Lys Asn Phe Phe Asn Tyr Leu
161                           170                                        180

Arg Asp Asn Lys Gly Tyr Asn Ala Ala Gly Gln Tyr Val Leu Ser Tyr Gln Phe Gly Thr
181                           190                                        200

Glu Pro Phe Thr Gly Ser Gly Thr Leu Asn Val Ala Ser Trp Thr Ala Ser Ile Asn
201                           210                                        219
```

# FIG. 15

# FIG. 16

| RANKING | POSITION 111 | POSITION 196 | POSITION 202 | | RANKING | POSITION 111 | POSITION 196 | POSITION 202 |
|---|---|---|---|---|---|---|---|---|
| 1 | L | L | A | | 26 | E | M | V |
| 2 | L | F | A | | 27 | L | I | T |
| 3 | L | I | A | | 28 | L | F | S |
| 4 | I | L | A | | 29 | I | F | T |
| 5 | I | F | A | | 30 | Y | F | A |
| 6 | L | L | P | | 31 | D | M | C |
| 7 | L | F | P | | 32 | I | I | P |
| 8 | H | W | V | | 33 | N | C | V |
| 9 | I | I | A | | 34 | L | L | S |
| 10 | I | F | P | | 35 | F | I | A |
| 11 | I | L | P | | 36 | D | V | C |
| 12 | Q | W | V | | 37 | K | M | A |
| 13 | L | L | T | | 38 | P | Y | A |
| 14 | D | M | A | | 39 | Y | Y | A |
| 15 | F | F | A | | 40 | E | V | V |
| 16 | H | Y | V | | 41 | M | F | A |
| 17 | F | L | A | | 42 | P | C | V |
| 18 | E | M | A | | 43 | N | W | V |
| 19 | L | I | P | | 44 | R | W | V |
| 20 | L | F | T | | 45 | K | W | V |
| 21 | G | S | W | | 46 | H | Y | A |
| 22 | E | M | C | | 47 | I | F | S |
| 23 | E | V | C | | 48 | D | I | A |
| 24 | I | L | T | | 49 | L | L | C |
| 25 | L | M | A | | 50 | E | I | A |

# FIG. 17

SPECIFIC HEAT RESISTANCE

SPECIFIC pH TOLERANCE

SPECIFIC EXPRESSION LEVEL

○ WILD-TYPE
● TOP 50 PREDICTED BY MACHINE LEARNING

# FIG. 18

| POSITION 111 | POSITION 196 | POSITION 202 | RANKING OF SCORE VALUE CALCULATED FROM MEASURED VALUE | RANKING OF PREDICTED SCORE VALUE | |
|---|---|---|---|---|---|
| | | | | COMBO | LightGBM |
| P | C | V | 1 | 42 | 95 |
| R | W | V | 2 | 44 | 45 |
| Q | W | V | 3 | 12 | 20 |
| M | F | A | 4 | 41 | 446 |
| L | F | A | 5 | 2 | 109 |
| I | F | A | 6 | 5 | 483 |
| D | M | A | 7 | 14 | 174 |
| N | C | V | 8 | 33 | 17 |
| H | Y | V | 9 | 16 | 23 |
| K | W | V | 9 | 45 | 97 |

## INTERNATIONAL SEARCH REPORT

| International application No. |
| --- |
| **PCT/JP2022/035925** |

**A.    CLASSIFICATION OF SUBJECT MATTER**

*C07K 16/00*(2006.01)i; *C12N 15/09*(2006.01)i; *C12N 15/10*(2006.01)i; *C12P 21/00*(2006.01)i; *C12P 21/08*(2006.01)i
    FI:    C12N15/10 200Z; C07K16/00; C12N15/09 Z ZNA; C12P21/00 Z; C12P21/08

According to International Patent Classification (IPC) or to both national classification and IPC

**B.    FIELDS SEARCHED**

Minimum documentation searched (classification system followed by classification symbols)

C07K16/00; C12N15/09; C12N15/10; C12P21/00; C12P21/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | SAITO, Y. et al. Machine-Learning-Guided Mutagenesis for Directed Evolution of Fluorescent Proteins. ACS Synth. Biol. 2018, vol. 7, no. 9, pp. 2014-2022<br>abstract, pp. 2015, 2017, 2020, fig. 1, 3, tables 2-3, etc. | 1-6, 8-14 |
| Y | abstract, pp. 2015, 2017, 2020, fig. 1, 3, tables 2-3, etc. | 7, 15, 16 |
| Y | 齋藤裕, 機械学習による生体分子の機能改良, JSBi Bioinformatics Review, 2020, vol. 1, no. 1, pp. 12-17<br>abstract, pp. 1314, (SAITO, Yutaka), non-official translation (Functional improvement of biomolecules by machine learning) | 7, 15, 16 |
| Y | WO 2019/198731 A1 (TOHOKU UNIVERSITY) 17 October 2019 (2019-10-17)<br>in particular, SEQ ID NO: 30 | 7, 15, 16 |
| X | in particular, SEQ ID NO: 30 | 7, 15, 16 |

☐ Further documents are listed in the continuation of Box C.    ☑ See patent family annex.

| | |
| --- | --- |
| *    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
| --- | --- |
| **02 December 2022** | **13 December 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/035925**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "Annex C/ST.25 text file format" should be read as "ST26 format."

Form PCT/ISA/210 (continuation of first sheet) (January 2015)

## INTERNATIONAL SEARCH REPORT

| International application No. |
| --- |
| **PCT/JP2022/035925** |

**Box No. III      Observations where unity of invention is lacking (Continuation of item 3 of first sheet)**

This International Searching Authority found multiple inventions in this international application, as follows:

The inventions in claims 1-15 and the invention in claim 16 share the common technical feature of a protein having amino acid variants.

However, said technical feature does not make a contribution over the prior art in light of the disclosures of documents 1-3 cited in the ISR. Thus, said technical feature cannot be said to be a special technical feature. Furthermore, there are no other identical or corresponding special technical features between these inventions.

Therefore, the claims include the following two inventions.
(Invention 1) The inventions as in claims 1-15
(Invention 2) The invention as in claim 16

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/035925**

| Patent document cited in search report | Publication date (day/month/year) | Patent family member(s) | Publication date (day/month/year) |
|---|---|---|---|
| WO 2019/198731 A1 | 17 October 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021035224 W
- WO 2005012877 A
- WO 2005013090 A
- WO 2019198731 A

### Non-patent literature cited in the description

- **SAITO et al.** Machine-Learning-Guided Mutagenesis for Directed Evolution of Fluorescent Proteins. *ACS Synth Biol.,* 2018, vol. 7 (9), 2014-2022
- **LIU et al.** Antibody complementarity determining region design using high-capacity machine learning. *Bioinformatics,* 2020, vol. 36 (7), 2126-2133
- **SAKA et al.** Antibody design using LSTM based deep generative model from phage display library for affinity maturation. *Scientific Reports,* 2021, vol. 11 (1), 5852
- **POREBSKI ; BUCKLE.** Consensus protein design. *Protein Engineering, Design & Selection,* 2016, vol. 29 (7), 245-251
- **STEIPE B. et al.** *J. Mol. Biol,* 1994, vol. 240 (3), 188-192
- **VAN WESTEN et al.** *J Cheminform.,* 2013, vol. 5, 41
- **UENO et al.** *Mater. Discov.,* 2016, vol. 4, 18-21
- **NIWA et al.** *PNAS,* 17 March 2009, vol. 106 (11), 4201-4206
- **ITO et al.** *Chemistry Letters,* 2021, vol. 50, 1867-187
- **NAKAZAWA et al.** *Appl Microbiol Biotechnol.,* 2009, vol. 83 (4), 649-57